Europäisches Patentamt

European Patent Office

Office européen des brevets

(19)

(11) Publication number: **0 473 155 A2**

# EUROPEAN PATENT APPLICATION

(12)

(21) Application number: **91114541.5**

(22) Date of filing: **29.08.91**

(51) Int. Cl.5: **C12Q 1/68**, C12P 19/34, //C07H21/04

(30) Priority: **30.08.90 US 575177**

(43) Date of publication of application:
**04.03.92 Bulletin 92/10**

(84) Designated Contracting States:
**AT BE CH DE ES FR GB IT LI NL**

(71) Applicant: **ABBOTT LABORATORIES**
**One Abbott Park Road**
**Abbott Park, Illinois 60064-3500(US)**

(72) Inventor: **Backman, Keith C.**
**200 Carlisle Road**

**Bedford, Massachusetts 01730(US)**
Inventor: **Carrino, John J.**
**5774 Constitution Street**
**Gurnee, Illinois 60031(US)**
Inventor: **Shimer, George H.**
**4751 Arsenal Street, Box 12**
**Watertown, Massachusetts 02172(US)**

(74) Representative: **Modiano, Guido et al**
**MODIANO, JOSIF, PISANTY & STAUB**
**Modiano & Associati Via Meravigli, 16**
**I-20123 Milano(IT)**

(54) **Controlled initial target-dependent production of templates for ligase chain reaction.**

(57) An improvement to the ligase chain reaction (LCR) method of selectively and geometrically ligating nucleic acid probes; before the LCR, a template-dependent controlled reaction increases the population of templates capable of supporting template-dependent ligation in LCR selectively in the presence of target nucleic acid, while substantially avoiding creation of any molecules capable of supporting template-dependent ligation in the absence of target nucleic acid. One way to control the initial reaction is to weight the reaction to create templates corresponding to only one of the two target strands, using e.g. template dependent ligation or polymerization. Another way to control the initial amplification is to create templates corresponding to both strands, but to prevent spurious creation of ligation templates by using a probe concentration low enough to avoid blunt end ligation or by using a method of amplification such as PCR that does not generate spurious ligation templates.

Fig. 1

EP 0 473 155 A2

EP 0 473 155 A2

This invention pertains to the detection of nucleic acid sequences; specifically, it pertains to target-dependent enhancement of signal relative to background in the detection of nucleic acid sequences by the Ligase Chain Reaction (LCR) technique described below.

An aspect of LCR involves a method for detecting the presence of specific nucleic acid sequences in samples. LCR features target-dependent ligation using two sets of nucleic acid probes. The members of the first set of probes are designed to hybridize to a target strand at abutting locations, in end to end fashion. The abutting probes thus aligned are ligated by template-dependent creation of a phosphodiester bond. The newly created molecule serves as a template (as does target complement if present) to mediate ligation of the second pair of probes to generate a new template for ligation of additional first pairs of probes, and so on.

As described below in more detail, the power of LCR derives from the ability of each set of ligated probes to act as a template for further ligation, thus providing ligation at an increasing (geometric) rate--i.e., an increasing number of molecules containing ligated target sequences is added by each cycle. LCR has been disclosed in EP-A-320 308. Because LCR generates a specific phosphodiester linkage and thereby amplifies the number of templates for creating such a linkage in further cycles, we may sometimes characterize LCR as an amplification technique. We do not mean to say that LCR amounts to amplification in the sense of synthesis of nucleic acid. LCR products have the sequence information of the probes provided, which is not necessarily identical to that of the target.

Ideally, the above-described template-dependent ligation occurs if and only if the template is present. In fact, however, template-independent ligation may occur as well. Template-independent ligation events create amplifiable products that can result in a background signal even in the absence of target. More specifically, LCR probe sets generally are in significant excess of the target DNA throughout most of the process. These excess complementary probes are free to hybridize to one another creating blunt end duplex DNA molecules which can be joined together by DNA ligase independently of target (albeit at substantially lower efficiencies than target mediated ligation), resulting in the generation of spurious templates that can then enable a chain reaction of template-dependent ligation and lead to a spurious "background" signal in LCR.

One method for controlling spurious background in LCR amplification involves modifying the ends of the probes to prevent ligation (including undesired blunt end ligation), and then reversing the modification in a template-dependent fashion. See EP-A-0 439 182 by Backman, Bond, Carrino and Laffler.

Wu, et al., in Polymerase Chain Reaction, Erlich, et al., Eds, (Cold Spring Harbor Laboratory Press, 1989), p. 233-236, describe allele specific amplification schemes. At one point, they suggest that LAR ("Ligase Amplification Reaction"--another name for LCR) may be used as an allele specific detection system for PCR enriched DNA sequences. It appears they propose to use PCR as an initial template controlled reaction, prior to LCR.

Wu, et al., Genomics, 4:560-569 (1989) review various amplification methods. They discuss both LAR (LCR) and PCR. In one variation, they describe a linear LCR reaction, employing just one set of ligatable probes (without their complementary set). They do not suggest, and apparently did not appreciate, the advantages to be obtained by combining an initial linear LCR with a subsequent full blown LCR.

Stated generally, we have discovered that the LCR can be improved by adding a first stage comprising a controlled template-dependent reaction in which the population of templates capable of supporting template-dependent ligation in LCR is increased while substantially avoiding template-independent (i.e. in the absence of target nucleic acid) creation of detrimental amounts of template molecules capable of supporting LCR. By "detrimental amounts" we mean amounts of spurious templates which limit sensitivity in comparison to LCR without the initial controlled reaction.

In one branch of the invention, the controlled reaction comprises template-dependent ligation controlled or weighted to substantially avoid detrimental blunt end ligation.

Specifically, in one preferred method of controlled ligation, the initial template-dependent ligation is heavily weighted to selectively produce ligation products having a sequence of or corresponding to (e.g., identical to or made by ligating probes hybridizable with) the target strand, over products having a sequence corresponding to the target complement strand. (We refer to the "two target strands" or "target and target complement" to designate not only the situation in which the original target is double stranded, but also recognizing that even where the target is initially single stranded, a ligation reaction will rapidly produce a ligation product which is hybridizable to at least part of the single-stranded target. Accordingly, we use the terms first and second target strand interchangeably with target and target complement in describing assays for targets which originally are present only as a single strand.)

In this preferred embodiment, one strand of the target is effectively the predominant template controlling ligation because effectively little or no means (i.e. reagents) are provided to use the target-

2

generated ligation products for further amplification. In the preferred extreme case, no means is provided for such secondary ligation at all and, in the controlled reaction, ligation is a substantially linear function of the number of ligation cycles. For that reason, we sometimes refer to the initial controlled ligation stage as linear amplification of the phosphodiester bonds or "linear preamplification".

The first reaction usually consists of 10-50 cycles, although up to 100 cycles or more may be performed.

When we say that the number of ligation events for sequences of one strand occurs "selectively" or that the reaction is "weighted" in favor of ligation events for one target strand, we mean that, in the first stage, product is produced preferentially from one target strand over the second target strand. This is done primarily by varying the ratio of reagents (probes) selectively operating on the two respective strands. The ratio may be controlled in a continuum from 1:1 (even amounts of reagents operating on each strand) to infinity (no reagents operating on one of the strands). In a preferred embodiment of the first branch of the invention, the controlled reaction features providing a set of the primary nucleic acid probes and ligating the primary probes in a template-dependent manner to create additional molecules hybridizable with the first target sequence. No "secondary" probes are provided.

Another preferred embodiment under the first branch of the invention takes advantage of the fact that DNA ligases can exhibit a decided preference for ligating nicked sites (i.e., for template-dependent ligation) over blunt-end ligation. Specifically, the desired template-dependent ligation is far more efficient, and its efficiency falls less rapidly with decreasing concentration than does the efficiency of blunt end ligation. Therefore, if the concentration of at least one set of probes is kept very low during the initial controlled stage, then the chance of blunt end ligation becomes vanishingly small. The concentration of the selected probe set may be reduced by as much as 100 fold, although about 10 fold appears to be sufficient. For example, we prefer a concentration for each probe of $10^{11}$ probes or less per standard reaction vessel (20-50uL) during the controlled reaction stage. This compares with standard LCR probe concentrations of about $10^{12}$ per 50 uL. While the rate of increase in ligated probes is reduced by reducing probe concentration, such a reduced rate can be tolerated in the initial controlled stage, which is performed to increase the number of templates initially present for standard LCR, rather than to provide directly detectable amounts of ligated species. In sum, we have taken advantage of the difference in relative efficiency of undesired blunt end ligation versus the desired template dependent ligation to produce LCR templates in a target-dependent manner, with a relatively minimal risk of blunt end ligation, thereby improving the result of the subsequent LCR reaction.

The two preferred embodiments of the first branch of the invention represent extremes of a continuum--i.e., standard concentrations of probes for one target strand with no complementary probes, on the one hand, and low concentrations of probes for both strands on the other hand. Those skilled in the art will appreciate that the invention includes points intermediate on this continuum i.e., using probes for both strands, but controlling concentration so that the population of probes for one strand is favored, and the likelihood of probe duplex formation and blunt end ligation is not detrimental. For example, fewer than $10^{11}$ molecules of probe per 50 uL vessel of at least one of the sets of probes may be present as described above.

A second branch of the invention features an initial controlled stage employing nucleic acid (preferably DNA) polymerization to generate the templates for LCR. Since polymerization is template dependent, creation of polymerization extension products generally will require the presence of the intended target sequence. To the extent that polymerization extension products are created from sites other than the intended target, such products will not significantly affect the subsequent LCR, because they will not serve as ligation templates. Accordingly, standard PCR, or polymerization weighted to selectively amplify one strand over the other, can serve as the initial controlled reaction. A significant advantage of using PCR as an initial controlled reaction to be followed by LCR is the resulting increased specificity over that achieved with PCR alone, by filtering out (eliminating) signal from spurious PCR extension products.

In one embodiment of the second branch of the invention, the controlled reaction comprises providing copies of a single primer nucleic acid sequence complementary to a portion of one strand of the target molecule (TM) 3' to the ligation point that will occur in subsequent LCR, together with a nucleic acid polymerase and a supply of nucleoside triphosphates (by which we mean to include and even prefer deoxribonucleoside triphosphates) suitable for template-dependent polymerization. Contrary to PCR, little, and preferably none, of the primer for the other strand customarily used in PCR is provided. This process has been described as "asymmetric PCR".

It will be recognized that this weighting of the polymerase-based controlled stage is a continuum just as it was for the ligase-based controlled stage. Those reagents are used in repeated cycles of first hybridizing the primer to the target nucleic acid sequence and then reacting the hybrid with the polymerase and nucleotide triphosphates to yield an extension product/template. Denaturation yields a single stranded extension product sequence hybridizable with a sequence of the first target strand. The cycle is then repeated a number of times.

The two controlled reactions described above (polymerization and ligation) can be performed serially. Preferably polymerization is performed first.

Also, when using either type of controlled reaction (polymerization or ligation), the reaction can be weighted to produce sequences corresponding to one strand as described above. A second parallel controlled reaction may be conducted in a separate vessel, the second reaction being weighted to produce sequences corresponding to the second target strand. The products of the two controlled amplification stages are then mixed so that the population of sequences of the two target strands subjected to LCR are approximately equal.

After any one of the controlled reaction embodiments, a standard LCR assay is used to achieve geometric amplification, in which probes with sequences corresponding to both target strand sequences are ligated, and each ligation product serves as a template for further ligation. Specifically, in the geometric stage, primary nucleic acid probes hybridize to adjacent portions of one target nucleic acid sequence and are ligated in a reaction that depends on the presence of a template (either the complement of the original target or ligation products created by ligation of probes in a manner dependent originally on the presence of the original target sequence as a template). Next, secondary nucleic acid probes hybridized to the ligated primary probes are ligated in a template-dependent manner to yield additional templates for ligation of the primary probes in a later cycle. Thus, the ligation products of the first amplification stage are submitted to geometric ligation through LCR.

A third aspect of the invention features a linear preamplification/LCR kit for performing the alternate (ligation-based) linear preamplification described above, comprising a DNA ligase, at least one of the sets of nucleic probes, a template-dependent polymerase, the above-described primer sequence, and a supply of nucleoside triphosphates suitable for polymerization by the polymerase. Preferably both the ligase and the polymerase are thermostable.

The invention permits a substantial increase in sensitivity by increasing the number of molecules containing a target sequence in a step that avoids the creation of spurious (target-independent) signal from blunt end ligation. The invention also provides the ability to control reagent concentrations and other preamplification variables such as cycle number to optimize final LCR signal sensitivity.

One way to evaluate the benefit of the invention is as follows. For the reasons given above, a spurious background signal almost invariably will develop in LCR after a sufficient number of cycles, even in the absence of the target sequence. The assay can be evaluated by the "window" (in terms of number of cycles) bounded by the number of LCR cycles that will reliably develop a signal in the presence of target and the number of cycles likely to generate a signal in the absence of target. According to the invention, through controlled template dependent reaction prior to LCR, the "window" is enlarged by reducing the number of LCR cycles necessary to develop a signal without substantially reducing the number of cycles at which a background signal appears in the absence of target.

Alternatively, the improvement of the invention may be evaluated in terms of improved sensitivity of an assay. In other words, compared to standard LCR, initial controlled amplification according to the invention improves the ability to distinguish samples containing target from samples without target.

Other features and advantages of the invention will be apparent to those skilled in the art from the following descriptions of the preferred embodiment.

## Brief Description of the Drawings

Fig. 1 is a diagram representing the linear preamplification steps in one embodiment of the invention.

Fig. 2 is a diagram of LCR following the linear preamplification of either Fig. 1 or Fig. 3.

Fig. 3 is a diagram representing the linear preamplification steps in a second embodiment of the invention.

Fig. 4 is a graph of the results of assays described in Example 4.

Fig. 5 is a graph of the results of assays described in Example 5.

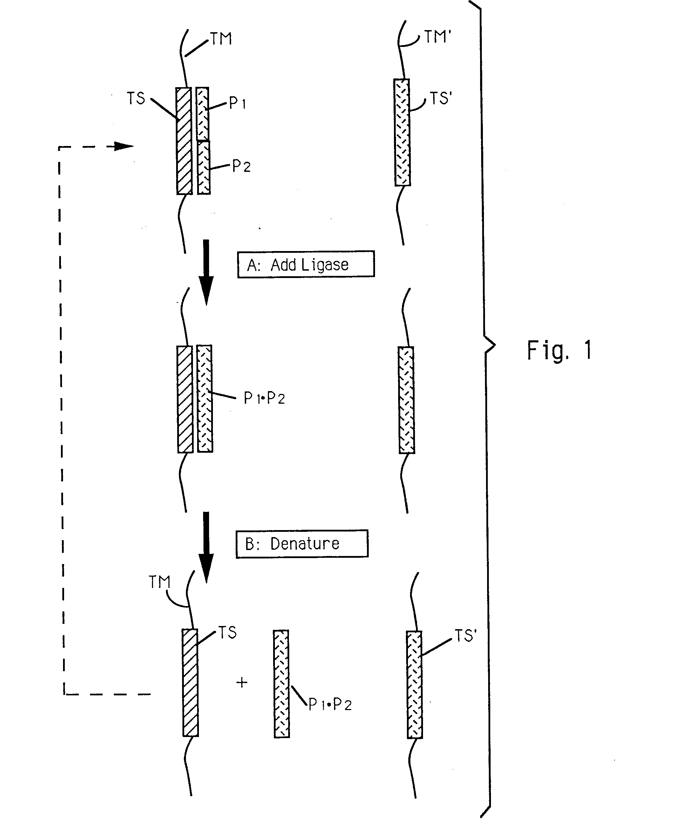

In Fig. 1, a single-stranded nucleic acid target molecule (e.g., DNA) ("TM") contains a known nucleic acid sequence target sequence ("TS"). (Note that TS need not be all of TM). The molecule TM may occur in the sample as a single stranded molecule; alternatively, the sample may contain a double stranded (hybridized) target molecule, in which case we arbitrarily denote one strand TM and the other TM'. Similarly TS' designates the sequence complementary to TS.

Controlled amplification according to the invention is depicted in Fig. 1 by target-dependent ligation of probes P1 and P2 hybridized to TS (step A, Fig. 1). By denaturation (step B, Fig. 1), the ligated entity P1.P2 is separated from TM. Note that this entity (P1.P2) cannot serve as a template for ligation of P1 and P2.

Each cycle comprises:

a) providing single stranded TS;

b) hybridizing P1 and P2 to TS;

c) ligating P1 to P2 in a template(TS)-dependent ligation reaction to yield a TS-P1.P2 duplex

d) denaturing the resulting TS-P1.P2 duplex yielding the original target molecule with sequence TS and the ligated complementary molecule P1.P2. In each cycle, there will be a particular yield of P1.P2, and that yield will be a function of the presence original target TS only, because no TS sequences are created during the linear phase. P1 and P2 effectively do not contribute to spurious background through blunt end (templated-independent) ligation because of the absence of complementary P1' and P2' sequences.

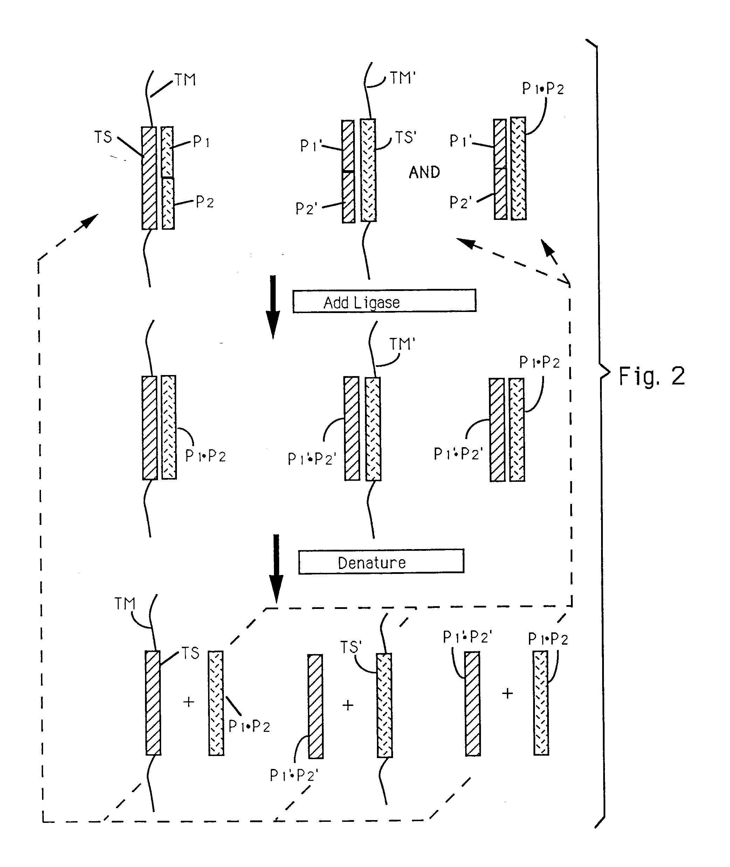

After the desired number of linear preamplification cycles, the LCR step (Fig. 2) involves addition of probes P1' and P2', in order to amplify the P1.P2 product of linear preamplification. Geometric LCR amplification in the presence of the P1.P2 product will yield a signal within a predictable number of cycles. In many situations, when LCR is applied directly to the target without initial controlled amplification, a greater number of LCR cycles would be required to yield that signal. In that way the invention substantially reduces the chance that undesired blunt end ligation products will be present in sufficient amount to yield an LCR signal in the cycle range producing a target-generated signal.

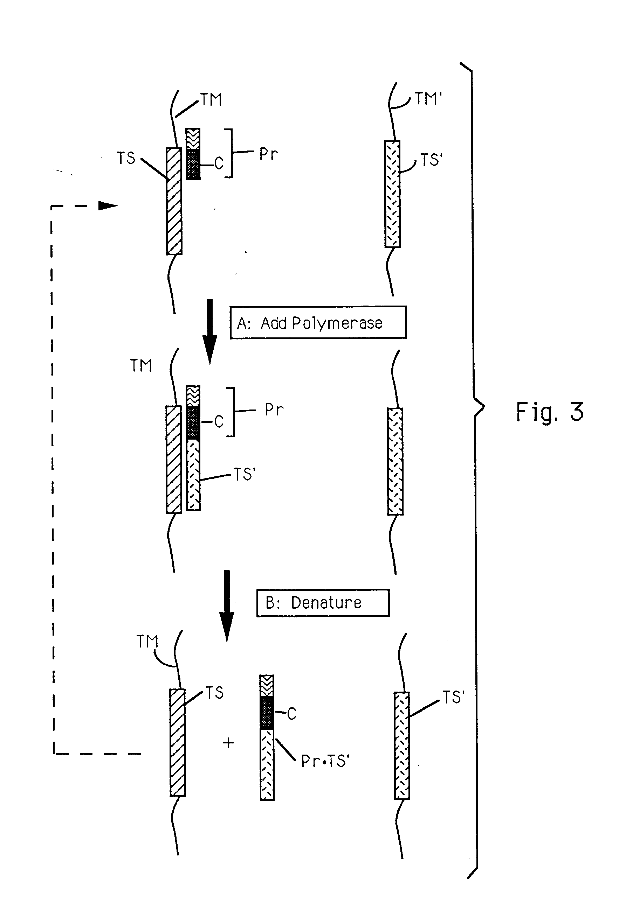

In Fig. 3 linear preamplification is achieved by polymerization, in contrast to the ligation shown in Fig. 1. Again, the target sequence TS of target molecule TM is used to produce additional copies of the complementary sequence TS'. A primer sequence Pr is supplied which includes a sequence C complementary to the 3' end of TS. Of course, the primer need only hybridize to the target molecule (TM) 3' to the ligation point that will occur in subsequent LCR. Sequence C is hybridized to TS, and polymerase and nucleotide triphosphates are added to extend sequence C in a template-dependent fashion, creating Pr.TS' containing an additional copy of TS', complementary to TS. As in the controlled ligation-based reaction, the result of multiple cycles is multiple copies of TS', but those copies do not themselves serve as template for further amplification during the linear stage, since a second primer is omitted.

Controlled preamplification techniques employing either ligase or polymerase enzymes are useful to increase the sensitivity of LCR. For example, controlled initial amplification increases the signal/noise ratio compared to a control that does not include controlled initial amplification. When evaluated in terms of improved sensitivity of an assay compared to a standard LCR assay, controlled amplification may improve the sensitivity by 10 fold or more thus enhancing the ability to distinguish samples containing the desired target from samples without the target.

In general, the controlled amplification is run for 10-100 cycles. The sample may then be heat treated at a temperature sufficient to destroy the activity of the enzyme present in the sample. If the initial controlled amplification is a ligased-based amplification, this step is optional. If necessary, the reaction mixture is then suitably adjusted (buffer, pH, etc.) in a manner known to those skilled in the art to support the LCR reaction.

Additional oligonucleotides are added to a concentration sufficient to serve as a complete LCR probe set, fresh ligase may be added, and LCR is performed on the target enriched sample. In general, oligonucleotides are used in the geometric phase in equimolar amounts at a final concentration of 10-100nM, more usually 30-100nM. Further considerations regarding oligonucleotides, reagents, and cycling conditions for the ligase linear amplification reaction are the same as those used for geometric LCR and are generally disclosed in EP-A-320 308. Conditions which can be used for controlled polymerase reactions are generally disclosed in Maniatis et al. Molecular Cloning (1982 Cold Spring Harbor); and Panet and Khorana (1974) J. Biol. Chem. 249:5213-5221. This polymerization differs from that described in U.S. patent Nos 4,683,202 and 4,683,195, in incorporated herein by reference, in that only one primer is used.

The following examples are provided to illustrate the invention, not to limit it. Various improvements and modifications may be practiced with the invention.

**Example 1: Controlled Amplification By Ligation**

The following duplex target DNA sequence is present in the commercially available vector PUC19. It is presented as a single strand for simplicity sake. The "-" in the sequence represents the intended point of ligation of the probes.

3'-...TTAAGCTCGA GCCATGGG-CC CCTAGGAGAT CTCAGCTGGA CGT...-5'

The following probes set was designed to hybridize to the target sequence for use in preamplification of the

target sequence:

```
A 5'-AATTCGAGCT CGGTACCC          SEQ ID No 1
B 5'-GGGGATCCTC TAGAGTCGACC TGCA  SEQ ID No 2
```

Primers A & B and their complements (A'&B') were prepared by standard methods using Applied Biosystems Model 380B. Primers A' and B were treated with polynucleotide kinase and ATP to render their 5' ends phosphorylated by the general method of Berger and Kimmel (1987) Guide to Molecular Cloning Techniques pp. 438 et seq. Primer A' was radioactively labeled at its 3' end by treatment with terminal transferase and $-^{32}$P-dCoTP (Cordecypin) by the general method of Tu and Cohen (1980) Gene 10:177-183.

Thermostable ligase was prepared according to the procedure described in EP-A-O 373 962. Samples were prepared which contained "1X LCR Buffer"--i.e., 50mM EPPS, pH 7.8, 100mM KCl, 10.0mM MgCl$_2$, 1.0mM dithiothreitol, 10mM NH$_4$Cl, 10ug/ml Bovine Serum Albumin--and 100uM NAD, 20ug/ml carrier DNA such as calf thymus DNA plus equimolar amounts of primers A and B (85nM each). Controlled amplification was performed on a sample containing 1000 target molecules in the presence of thermostable ligase. A single cycle encompasses the following steps and is the same for either linear or geometric ligase-mediated amplification.

a) Heat to 90°C for 1 minute to denature the DNA

b) incubate at 50°C for 1 minute to allow annealing and ligation of adjacent probes.

After 50 cycles, the reaction was stopped and ligase was denatured by boiling for 10 min. To the "preamplified" sample were added the other two probes (A' & B'; 80nM each) of the LCR probe set and new ligase, and cycling was resumed for an additional 20-50 cycles. Samples were withdrawn at various times and analyzed by polyacrylamide gel electrophoresis and autoradiography. As controls, standard LCR reactions were performed using the four probes and either zero or 1000 targets. Samples were withdrawn and analyzed as for the preamplified reaction.

The control sample without target sequence produces signal with characteristic kinetics, and the sample with 1000 target sequences (but no controlled amplification) produces signal which has very similar kinetics. The signal in the target-containing sample subjected to initial controlled amplification, however, appeared at least 4 to 6 cycles earlier in the LCR (geometric amplification) procedure than the others. Thus, by beginning with controlled amplification, 1000 targets can be readily distinguished from background in a case where such distinction is difficult without preamplification. This allows identification of numbers of targets not reliably identifiable without linear pre-amplification, and represents an improved sensitivity.

**Example 2: Controlled Amplification by Polymerization**

Linear preamplification by polymerization also improves the sensitivity of the LCR assay. Accordingly, Example 2 features the use of the target and probe sequences given in Example 1. To a sample containing 1000 targets, a single oligonucleotide probe, A, is added. Thermostable DNA polymerase, buffer, and dNTPs are added according to the method described in Maniatis et al., cited above; see also the other references cited above regarding polymerization. Fifty hybridization, extension, and denaturation cycles are performed; the reaction is then stopped and the polymerase activity is destroyed by boiling for 10 minutes.

The entire preamplified sample is ethanol precipitated and centrifuged, residual salts removed by ethanol wash and the precipitate dried. The precipitate is then resuspended in the standard LCR reaction mix, probes A, B, A', B' (80nM for each probe) are added, thermostable ligase is added, and LCR is performed. Controls containing 1000 or zero targets are also run.

**Example 3: Serial Controlled Amplification**

Using the target and probe sequences given in Example 1, a single oligonucleotide probe, A, is added to a sample containing 1000 targets. Polymerase-mediated preamplification is performed as in Example 2 including precipitation, washing, resuspension in LCR buffer and addition of ligase enzyme. As opposed to Example 2, only probes A' and B' are added, probe B is omitted. In the absence of probe B, geometric amplification cannot occur and thus a second linear amplification step is initiated. The linear reaction is performed for 50 cycles at which point the reaction is stopped as in Example 1, and fresh thermostable ligase and probe B are added and the geometric amplification is performed for 20-50 additional cycles. Controls may be run as in the previous Examples.

**Example 4: Controlled Amplification with Detection on the IMx® instrument**

Duplex DNA having the following sequence (adapted from the L1 region of Human Papilloma Virus Type 16) was used as a target to study the linear preamplification process. The sequence is presented as a single strand for simplicity with a hyphen designating the ligation site:

5'-AGGTTGTAAG CACGGATGAA TATGT-TGCAC GCACAAACAT ATATTATCAT G-3'

The following probe set was designed to hybridize to the above target sequence for use in preamplifiction of the target sequence. The probes were synthesized by standard techniques (Fl = fluorescein and Bio = biotin).

```
179.1    Fl-AAGTTGTAAG  CACGGATGAA  TATGT-3'        SEQ  ID  No  3
179.2    5'-ACATATTCAT  CCGTGCTTAC  AACT-Fl'        SEQ  ID  No  4
179.3    5'-TGCACGCACA  AACATATATA  TTACA-Bio       SEQ  ID  No  5
179.4    Bio-ATGATAATAT  ATGTTTGTGC  GTGCA-3'        SEQ  ID  No  6
```

The 5' end of probe 179.1 was haptenated with fluorescein for use in the IMx MEIA assay as follows. A solution of 15 uL of 0.9 $A_{260}$ Unit/uL of oligo 5' XAAGTTGTAAGCACGGATGAATATGT-3' (ID No 3), where X is Aminomodifier II(TM) (Clontech), was treated with 4 mg of fluorescein isothiocyanate (FITC, Kodak) dissolved in 485 uL of sodium borate buffer, pH 9.2, at room temperature for 15h in the dark. The excess FITC was removed on a NAP-5 column (Pharmacia), and the eluate was concentrated to a volume of 100 uL on a Speedvac(TM) (Savant Instruments). The solution was diluted to 200 uL with formamide, and the sample was separated by electrophoresis at 40 W constant power on a 1.5 mm thick, 12% acrylamide/8M urea gel. The electrophoresis was stopped after 5h, and the product band on the gel was visualized with long-wave UV shadowing. The product had a lower mobility than unlabeled starting material. The excised FITC-oligo band was extracted overnight with 3 ml of 1.0 M triethylammonium acetate, and the aqueous extracts were lyophilized. Reconstitution of the residue in distilled water was followed by NAP-5 desalting. After haptenation, the probe was diluted to 1012 molecules per uL in Tris-EDTA buffer (10mM Tris, 1mM EDTA).

The 3' end of probe 179.2 (ID No 4) was haptenated with fluorescein as described above, using 3'-amino CPG (Glen Research) in place of 5' Aminomodifier II(TM). The probe is treated with polynucleotide kinase and diluted to $10^{12}$ molecules per uL.

The 3' end of probe 179.3 was haptenated as follows: A solution of 35uL of 0.378 $A_{260}$ Unit/uL of oligo 5'-TGCACGCACAAACATATATTATCAX-3', (ID No 5) where X is 3'-amino CPG (CPG = controlled pore glass; Glen Research), was treated with 10 mg of Biotin-(aminocaproyl)2-NHS active ester (Clontech) in 215/250 uL 100 mM pH 7.5 phosphate/DMF at room temperature for 15h. Workup and electrophoresis as in the fluorescein case gave a product band on short wave UV shadowing which migrated the equivalent of 4 bases slower than the starting material. Excision and extraction, followed by desalting as in the case of fluorescein. The probe was treated with polynucleotide kinase and ATP to phosphorylate the 5' end, and it was diluted to $10^{12}$ molecules per uL.

The 5' end of probe 179.4 (ID No 6) was haptenated as described above and diluted to $10^{12}$ molecules per uL.

Each linear preamplification was run in a 50uL volume as follows:

|  | (uL) |
|---|---|
| dH2O | 32.75 |
| 5X LCR Buffer | 10.0 |
| 10mM NAD | 0.5 |
| 179.1 ($10^{12}$ molecules per uL) | 0.5 |
| 179.3 ($10^{12}$ molecules per uL) | 0.75 |
| target (varied concentrations) | 1.5 |
| 1X ligase (from 2 to $10 \times 10^6$ units where 1 unit seals 1uM nicked DNA per minute) | 1.0 |
|  | 50.0 |

Where possible, reaction volumes were created from larger, pooled volumes that were divided into aliquots. Each individual reaction was overlayed with 30uL of mineral oil.

Each reaction aliquot (minus the ligase) was subjected to 100°C for 3 minutes to denature the target, followed by 85°C for 30 sec and 50°C for 20 sec. Then 1uL of ligase was added. The preamplification reaction was run for 50 thermal cycles (30 secs at 85°C followed by 20 secs at 50°C).

The LCR geometric amplification phase was accomplished by adding a 3uL aliquot of a pool consisting of:

| dH$_2$O | 1.75uL |
|---|---|
| 179.2 ($10^{12}$ molecules/uL) | 0.75uL |
| 179.4 ($10^{12}$ molecules/uL) | 0.5uL |

LCR reactions were run for 30 cycles. Appropriate controls were run.

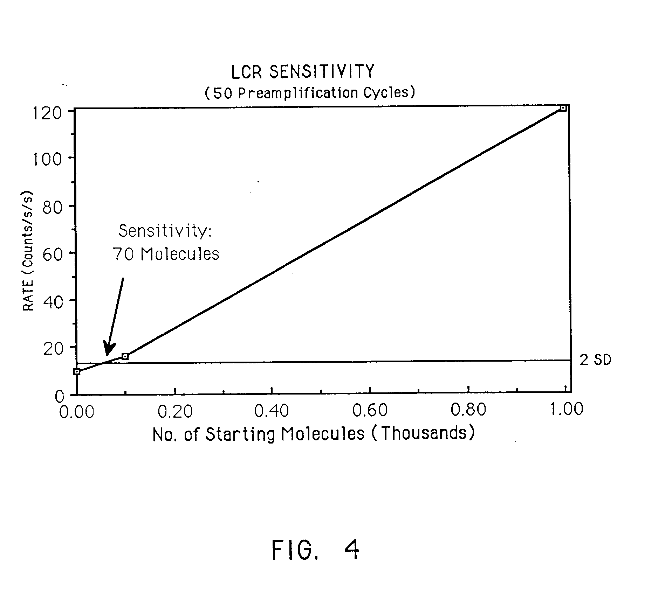

A comparison of LCR under comparable conditions with and without linear preamplification showed that linear preamplification significantly increased sensitivity as shown in Fig. 4.

**Example 5: Reduced Initial Probe Concentration**

The following example illustrates template-dependent ligation using reduced probe concentration as a method for controlling blunt end ligation.

The following two stock solutions were prepared.

STOCK SOLUTION #1

6.3 uL 10x LCR buffer
4.5 uL 10 mM NAD
6.2 uL H$_2$0
4.5 uL each probe at $10^{11}$ oligonucleotides/uL
10.0 uL CoT$^{32}$P-(Cordecypin) labelled probe.

STOCK SOLUTION #2

2.0 uL 10x LCR buffer
4.5 uL each probe at $10^{12}$ oligonucleotides/uL.

An aliquot of 10 uL of SOLUTION #1 was added to each of four(4) 0.65 ml Eppendorf tubes together with 3.0 uL of background DNA with or without target to each tube. LCR reaction was initiated and continued for 20 cycles. The tube was spun briefly in a centrifuge and returned to 90°C. 4.0 uL of SOLUTION #2 was added to each tube and temperature cycling was continued as with standard LCR.

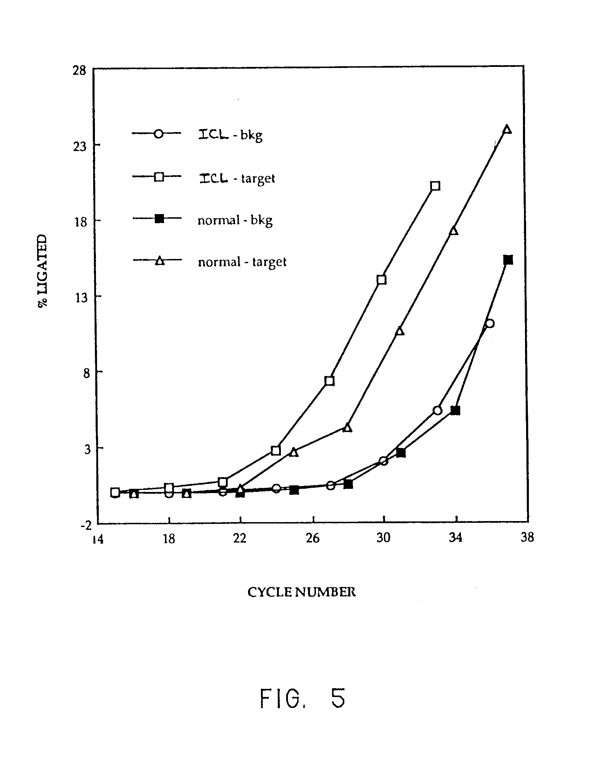

Fig. 5 depicts the results of the control (standard LCR) and of the use of an initial controlled (low probe concentration) ligation ("ICL").

## Example 6: PCR as the Initial Controlled Reaction

A segment of the cystic fibrosis (CF) gene was amplified by PCR from genomic DNA samples obtained from several CF families using primers A and B (Table 1). PCR was performed for 30 cycles. Each cycle consisted of a 60 sec incubation at 94°C, a 43 sec incubation at 62°C, and a 120 sec incubation at 72°C.

Following PCR, reaction products were diluted 1:200 with water and 1uL aliquots were used as target for LCR reactions with probe sets specific for the normal CF allele and the major defective CF allele which contains a three nucleotide deletion (Riordan, J.R. et al. Science **245**:1066 (1989); Kerem, B. et al. Science **245**:1073 1989). LCR probes used for amplification of the normal and deleted CF alleles are listed in Table 1. Probes C', E', and D' were phosphorylated as in Example 1, above. LCR was performed for 25 cycles. Each cycle consisted of a 30 sec incubation at 85°C and a 20 sec incubation at 50°C. For identification of the normal allele, oligonucleotides C' and D were used at $7.5 \times 10^{11}$ molecules/reaction and oligonucleotides E and D' at $5 \times 10^{11}$ molecules/reaction. For the deleted allele, oligonucleotides C' and D were used at $7.5 \times 10^{11}$ molecules/reaction and oligonucleotides E and D' at $5 \times 10^{11}$ molecules/reaction. Reaction volume in each case was 50uL. Following LCR, reaction products were analyzed by the IMx® MEIA assay. Selected representative results are shown in Table 2. Although all patients were "PCR positive", LCR distinguished those who carried the CF allele from those who did not.

This Example demonstrates the improvement of the invention of the invention if the normal allele is viewed as "true" target and the deleted allele is viewed as spurious extension.

## Table 1

PCR Primers:
A:   5'-GTTTTCCTGG ATTATGCCTG GGCAC-3'            SEQ  ID No 7
B:   5'-GTTGGCATGC TTTGATGACG CTTC-3'             SEQ  ID No 8

LCR Probes:
   Normal Allele
C:   Fl-CACCATTAAA GAAAATATCA TCTT-3'             SEQ  ID No 9
C':  5'-AAGATGATAT TTTCTTTAAT GGTGC-Fl            SEQ  ID No 10
D:   5'-TGGTGTTTCC TATGATGAAT ATAGA-Bio           SEQ  ID No 11
D':  BIO-CTATATTCAT CATAGGAAAC ACCA-3'            SEQ  ID No 12

   Deleted Allele
E:   Fl-TGGCACCATT AAAGAAAATA TCAT-3'             SEQ  ID No 13
E':  5'-ATGATATTTT CTTTAATGGT GCCAG-Fl            SEQ  ID No 14
D:   5'-TGGTGTTTCC TATGATGAAT ATAGA-Bio           SEQ  ID No 15
D':  Bio-CTATATTCAT CATACGAAAC ACCA-3'            SEQ  ID No 16

## Table 2

| | IMx Signal (counts/sec/sec) | |
| | Normal | Deleted |
|---|---|---|
| No target | 19 | 97 |
| patient 1 | 1826 | 107 |
| patient 2 | 1973 | 1997 |
| patient 3 | 24 | 1946 |

Other embodiments are within the following claims. For example, the low probe concentration controlled amplification of Example 5 may be combined with one or more of the weighted amplification stages

described above.

## Claims

1. A method for performing a ligase chain reaction (LCR) on target nucleic acid in a sample, wherein a first template-dependent controlled reaction, prior to said LCR, increases the population of molecules capable of participating as templates in said LCR selectively in the presence of said target nucleic acid, while substantially avoiding creation of detrimental amounts of template molecules capable of participating in said LCR in the absence of said target nucleic acid, characterized in that said template-dependent controlled reaction comprises:
    providing a first set of ligatable nucleic acid probes capable of hybridizing to a target strand, in substantial excess over a second set of ligatable probes (if any) capable of hybridizing to complement of the target strand or to ligated first probes; and ligating said members of said first set of probes in a template-dependent manner, the second set of probes (if any) being provided in a concentration low enough to substantially avoid detrimental ligation independent of target.

2. The method of claim 1 in which substantially no amplification products containing a sequence of the target complement strand are generated in said controlled reaction, and substantially the only template for said first controlled reaction is said target nucleic acid.

3. The method of claim 2 further comprising a parallel template-dependent controlled reaction performed in a separate vessel from the vessel used for said first controlled reaction, said parallel controlled

14

reaction comprising a reaction weighted to generate products containing a sequence corresponding to said target complement strand selectively with substantially no products containing a sequence corresponding to the target strand and said method further comprising mixing products of said first controlled reaction with products of said parallel controlled reaction, and performing LCR on said mixture.

4. The method of claim 1 in which the template-dependent controlled reaction comprises a ligase chain reaction using a concentration for at least one of the sets of LCR probes low enough to substantially eliminate blunt end ligation.

5. The method of claim 4 wherein the concentration of one set of probes exceeds the concentration of the other set of probes by about 10 fold.

6. The method of claim 4 in which the reaction vessel has a volume of 20 to 50 uL and the concentration of both probes for said one set of LCR probes is no more than $10^{11}$ molecules.

7. The method of claim 1 in which the controlled reaction comprises a template-dependent polymerization stage and a template-dependent ligation stage, said ligation stage comprising template-dependent ligation substantially without creation of detrimental amounts of blunt end ligation products.

8. The method of claim 7 in which said polymerization stage is performed first and said ligation stage is performed second.

Fig. 1

Fig. 2

Fig. 3

LCR SENSITIVITY
(50 Preamplification Cycles)

FIG. 4

FIG. 5


APPENDIX
SEQUENCE LISTING

(1) GENERAL INFORMATION:

    (i) APPLICANT:  Abbott Laboratories

   (ii) TITLE  OF INVENTION:  CONTROLLED INITIAL TARGET-DEPENDENT
       PRODUCTION OF TEMPLATES FOR LIGASE CHAIN REACTION

   (iii)  NUMBER OF SEQUENCES:  16

  (viii)  ATTORNEY/AGENT INFORMATION:
          (A) NAME:  MODIANO, JOSIF, PISANTY & STAUB
          (C) REFERENCE/DOCKET NUMBER:  23319/GM/fh

(2)  INFORMATION FOR SEQ ID NO: 1:

    (i)  SEQUENCE CHARACTERISTICS:
        (A) LENGTH:  17
        (B) TYPE:  nucleic acid
        (C) STRANDEDNESS:  single
        (D) TOPOLOGY:  linear

    (ii)  MOLECULE TYPE:  Other nucleic acid (synthetic DNA)

    (xi)  SEQUENCE DESCRIPTION: SEQ ID NO: 1:

AATTCGAGCT CGGTACCC                                    **17**

(3)  INFORMATION FOR SEQ ID NO: 2:

    (i)  SEQUENCE CHARACTERISTICS:
        (A) LENGTH:  24
        (B) TYPE:  nucleic acid
        (C) STRANDEDNESS:  single
        (D) TOPOLOGY:  linear

    (ii)  MOLECULE TYPE:  Other nucleic acid (synthetic DNA)

    (xi)  SEQUENCE DESCRIPTION: SEQ ID NO: 2:

GGGGATCCTC TAGAGTCGACC TGCA                           **24**

(4)  INFORMATION FOR SEQ ID NO: 3:

    (i)  SEQUENCE CHARACTERISTICS:
        (A) LENGTH:  25
        (B) TYPE:  nucleic acid
        (C) STRANDEDNESS:  single
        (D) TOPOLOGY:  linear

(ii)    MOLECULE TYPE:  Other nucleic acid (synthetic DNA)

(xi)    SEQUENCE DESCRIPTION: SEQ ID NO: 3:

AAGTTGTAAG CACGGATGAA TATGT                                    25

(5)   INFORMATION FOR SEQ ID NO: 4:

    (i)    SEQUENCE CHARACTERISTICS:
           (A) LENGTH:   24
           (B) TYPE:  nucleic acid
           (C) STRANDEDNESS:  single
           (D) TOPOLOGY:  linear

(ii)    MOLECULE TYPE:  Other nucleic acid (synthetic DNA)

(xi)    SEQUENCE DESCRIPTION: SEQ ID NO: 4:

ACATATTCAT CCGTGCTTAC AACT                                     24

(6)   INFORMATION FOR SEQ ID NO: 5:

    (i)    SEQUENCE CHARACTERISTICS:
           (A) LENGTH:   25
           (B) TYPE:  nucleic acid
           (C) STRANDEDNESS:  single
           (D) TOPOLOGY:  linear

(ii)    MOLECULE TYPE:  Other nucleic acid (synthetic DNA)

(xi)    SEQUENCE DESCRIPTION: SEQ ID NO: 5:

TGCACGCACA AACATATATA TTACA                                    25

(7)   INFORMATION FOR SEQ ID NO: 6:

    (i)    SEQUENCE CHARACTERISTICS:
           (A) LENGTH:   25
           (B) TYPE:  nucleic acid
           (C) STRANDEDNESS:  single
           (D) TOPOLOGY:  linear

(ii)    MOLECULE TYPE:  Other nucleic acid (synthetic DNA)

(xi)    SEQUENCE DESCRIPTION: SEQ ID NO: 6:

ATGATAATAT ATGTTTGTGC GTGCA                                    25

(8)   INFORMATION FOR SEQ ID NO: 7:

(i)   SEQUENCE CHARACTERISTICS:
          (A) LENGTH:   25
          (B) TYPE:   nucleic acid
          (C) STRANDEDNESS:   single
          (D) TOPOLOGY:   linear

(ii)   MOLECULE TYPE:   Other nucleic acid (synthetic DNA)

(xi)   SEQUENCE DESCRIPTION: SEQ ID NO: 7:

GTTTTCCTGG ATTATGCCTG GGCAC          25


(9)   INFORMATION FOR SEQ ID NO: 8:

(i)   SEQUENCE CHARACTERISTICS:
          (A) LENGTH:   24
          (B) TYPE:   nucleic acid
          (C) STRANDEDNESS:   single
          (D) TOPOLOGY:   linear

(ii)   MOLECULE TYPE:   Other nucleic acid (synthetic DNA)

(xi)   SEQUENCE DESCRIPTION: SEQ ID NO: 8:

GTTGGCATGC TTTGATGACG CTTC                                    24


(10)   INFORMATION FOR SEQ ID NO: 9:

(i)   SEQUENCE CHARACTERISTICS:
          (A) LENGTH:   24
          (B) TYPE:   nucleic acid
          (C) STRANDEDNESS:   single
          (D) TOPOLOGY:   linear

(ii)   MOLECULE TYPE:   Other nucleic acid (synthetic DNA)

(xi)   SEQUENCE DESCRIPTION: SEQ ID NO: 9:

CACCATTAAA GAAAATATCA TCTT                                    24


(11)   INFORMATION FOR SEQ ID NO: 10:

(i)   SEQUENCE CHARACTERISTICS:
          (A) LENGTH:   25
          (B) TYPE:   nucleic acid
          (C) STRANDEDNESS:   single
          (D) TOPOLOGY:   linear

(ii)   MOLECULE TYPE:   Other nucleic acid (synthetic DNA)

(xi)   SEQUENCE DESCRIPTION: SEQ ID NO: 10:

AAGATGATAT TTTCTTTAAT GGTGC                                        25

(12)    INFORMATION FOR SEQ ID NO: 11:

    (i)    SEQUENCE CHARACTERISTICS:
        (A) LENGTH:   25
        (B) TYPE:  nucleic acid
        (C) STRANDEDNESS:  single
        (D) TOPOLOGY:  linear

    (ii)   MOLECULE TYPE:  Other nucleic acid (synthetic DNA)

    (xi)   SEQUENCE DESCRIPTION: SEQ ID NO: 11:

TGGTGTTTCC TATGATGAAT ATAGA                                        25

(13)    INFORMATION FOR SEQ ID NO: 12:

    (i)    SEQUENCE CHARACTERISTICS:
        (A) LENGTH:   24
        (B) TYPE:  nucleic acid
        (C) STRANDEDNESS:  single
        (D) TOPOLOGY:  linear

    (ii)   MOLECULE TYPE:  Other nucleic acid (synthetic DNA)

    (xi)   SEQUENCE DESCRIPTION: SEQ ID NO: 12:

CTATATTCAT CATAGGAAAC ACCA                                         24

(14)    INFORMATION FOR SEQ ID NO: 13:

    (i)    SEQUENCE CHARACTERISTICS:
        (A) LENGTH:   24
        (B) TYPE:  nucleic acid
        (C) STRANDEDNESS:  single
        (D) TOPOLOGY:  linear

    (ii)   MOLECULE TYPE:  Other nucleic acid (synthetic DNA)

    (xi)   SEQUENCE DESCRIPTION: SEQ ID NO: 13:

TGGCACCATT AAAGAAAATA TCAT                                         24

(15)    INFORMATION FOR SEQ ID NO: 14:

    (i)    SEQUENCE CHARACTERISTICS:
        (A) LENGTH:   25
        (B) TYPE:  nucleic acid
        (C) STRANDEDNESS:  single
        (D) TOPOLOGY:  linear

(ii)  MOLECULE TYPE:  Other nucleic acid (synthetic DNA)

(xi)  SEQUENCE DESCRIPTION: SEQ ID NO: 14:

ATGATATTTT CTTTAATGGT GCCAG                                    25


(16)  INFORMATION FOR SEQ ID NO: 15:

    (i)  SEQUENCE CHARACTERISTICS:
         (A) LENGTH:  25
         (B) TYPE:  nucleic acid
         (C) STRANDEDNESS:  single
         (D) TOPOLOGY:  linear

(ii)  MOLECULE TYPE:  Other nucleic acid (synthetic DNA)

(xi)  SEQUENCE DESCRIPTION: SEQ ID NO: 15:

TGGTGTTTCC TATGATGAAT ATAGA                                    25


(17)  INFORMATION FOR SEQ ID NO: 16:

    (i)  SEQUENCE CHARACTERISTICS:
         (A) LENGTH:  24
         (B) TYPE:  nucleic acid
         (C) STRANDEDNESS:  single
         (D) TOPOLOGY:  linear

(ii)  MOLECULE TYPE:  Other nucleic acid (synthetic DNA)

(xi)  SEQUENCE DESCRIPTION: SEQ ID NO: 16:

CTATATTCAT CATACGAAAC ACCA                                     24